# EUROPEAN PATENT APPLICATION

(11) **EP 1 375 678 A2**
(43) Date of publication of application: **02.01.2004**
(21) Application number: 03253764.9
(22) Date of filing: 13.06.2003
(51) Int. Cl.: C12Q 1/68

(54) **Methods and compositions for performing array based assays**

(30) Priority: 14.06.2002 US 388947 P
(71) Applicant: Agilent Technologies, Inc., Palo Alto, CA 94306 (US)
(72) Inventor: Amorese, Douglas A., Los Altos, CA 94022 (US); Caren, Michael P., Palo Alto, CA 94303 (US); Schleifer, Arthur, Portola Valley, CA 94028 (US); Holcomb, Nelson R., Palo Alto, CA 94301 (US)
(74) Representative: Tollett, Ian

(57) **Abstract**

Methods of performing array-based assays are provided. A feature of the subject methods is that a sample is initially contacted with a first substrate, i.e., a backing element, to produce a substrate supported sample. Next, the resultant substrate supported sample is contacted with an array of at least two different ligands, and the presence of any resultant surface bound binding complexes is detected. Also provided are backing elements, as well as kits and systems, for use in practicing the subject methods. The subject methods and compositions find use in any array based application, including genomic and proteomic applications.

## Description

This application claims priority to the filing date of the United States Provisional Patent Application Serial No. 60/388,947 filed June 14, 2002; the disclosure of which is herein incorporated by reference.

The present application relates to a method of assaying a sample, a backing element for use in an array assay and to systems and kits for performing assays. The field of this invention is ligand, e.g., biopolymeric, arrays.

Array assays between surface bound binding agents or probes and target molecules in solution may be used to detect the presence of particular biopolymers. The surface-bound probes may be oligonucleotides, peptides, polypeptides, proteins, antibodies or other molecules capable of binding with target molecules in solution. Such binding interactions are the basis for many of the methods and devices used in a variety of different fields, e.g., genomics (in sequencing by hybridization, SNP detection, differential gene expression analysis, identification of novel genes, gene mapping, finger printing, etc.) and proteomics.

One typical array assay method involves biopolymeric probes immobilized in an array on a substrate such as a glass substrate or the like. A solution containing analytes that bind with the attached probes is placed in contact with the substrate, covered with another substrate to form an assay area and placed in an environmentally controlled chamber such as an incubator or the like. Usually, the targets in the solution bind to the complementary probes on the substrate to form a binding complex. The pattern of binding by target molecules to biopolymer probe features or spots on the substrate produces a pattern on the surface of the substrate and provides desired information about the sample. In most instances, the target molecules are labeled with a detectable tag such as a fluorescent tag, chemiluminescent tag or radioactive tag. The resultant binding interaction or complexes of binding pairs are then detected and read or interrogated, for example by optical means, although other methods may also be used. For example, laser light may be used to excite fluorescent tags, generating a signal only in those spots on the biochip that have a target molecule and thus a fluorescent tag bound to a probe molecule. This pattern may then be digitally scanned for computer analysis.

A problem experienced with many array assay formats is background, which can adversely impact that data that is obtained from the array assay. One source of background is the presence of background causing materials, e.g., unincorporated labeling reagents, etc., in the sample that is contacted with the array surface. While various protocols have been developed to process samples prior to array contact in order to remove background causing materials, such protocols tend to be insufficiently effective and/or resource intensive. Thus, there continues to be an interest in the development of new array based assays protocols in which background is kept to a minimum.

Methods of performing array-based assays are provided. A feature of the subject methods is that a sample is initially contacted with a first substrate, i.e., a backing element, to produce a substrate supported sample. Next, the resultant substrate supported sample is contacted with an array of at least two different ligands, and the presence of any resultant surface bound binding complexes is detected. Also provided are backing elements, as well as kits and systems, for use in practicing the subject methods. The subject methods and compositions find use in any array based application, including genomic and proteomic applications.

A number of preferred embodiments of the invention will now be described with reference to the drawings, in which:-
Figure 1 shows an exemplary substrate carrying an array, such as may be used in the devices of the subject invention.
Figure 2 shows an enlarged view of a portion of Figure 1 showing spots or features.
Figure 3 is an enlarged view of a portion of the substrate of Figure 1.

### DEFINITIONS

Unless defined otherwise, all technical and scientific terms used herein have the same meaning as commonly understood by one of ordinary skill in the art to which this invention belongs. Still, certain elements are defined below for the sake of clarity and ease of reference.

The term "biomolecule" means any organic or biochemical molecule, group or species of interest that may be formed in an array on a substrate surface. Exemplary biomolecules include peptides, proteins, amino acids and nucleic acids.

The term "peptide" as used herein refers to any compound produced by amide formation between a carboxyl group of one amino acid and an amino group of another group.

The term "oligopeptide" as used herein refers to peptides with fewer than about 10 to 20 residues, *i.e.* amino acid monomeric units.

The term "polypeptide" as used herein refers to peptides with more than 10 to 20 residues.

The term "protein" as used herein refers to polypeptides of specific sequence of more than about 50 residues.

The term "nucleic acid" as used herein means a polymer composed of nucleotides, e.g., deoxyribonucleotides or ribonucleotides, or compounds produced synthetically (e.g. PNA as described in U.S. Patent No. 5,948,902 and the references cited therein) which can hybridize with naturally occurring nucleic acids in a sequence specific manner analogous to that of two naturally occurring nucleic acids, e.g., can participate in Watson-Crick base pairing interactions.

The terms "nucleoside" and "nucleotide" are intended to include those moieties that contain not only the known purine and pyrimidine base moieties, but also other heterocyclic base moieties that have been modified. Such modifications include methylated purines or pyrimidines, acylated purines or pyrimidines, or other heterocycles. In addition, the terms "nucleoside" and "nucleotide" include those moieties that contain not only conventional ribose and deoxyribose sugars, but other sugars as well. Modified nucleosides or nucleotides also include modifications on the sugar moiety, e.g., wherein one or more of the hydroxyl groups are replaced with halogen atoms or aliphatic groups, or are functionalized as ethers, amines, or the like.

The terms "ribonucleic acid" and "RNA" as used herein refer to a polymer composed of ribonucleotides.

The terms "deoxyribonucleic acid" and "DNA" as used herein mean a polymer composed of deoxyribonucleotides.

The term "oligonucleotide" as used herein denotes single stranded nucleotide multimers of from about 10 to 100 nucleotides and up to 200 nucleotides in length.

The term "polynucleotide" as used herein refers to single or double stranded polymer composed of nucleotide monomers of generally greater than 100 nucleotides in length.

A "biopolymer" is a polymeric biomolecule of one or more types of repeating units. Biopolymers are typically found in biological systems and particularly include polysaccharides (such as carbohydrates), peptides (which term is used to include polypeptides and proteins) and polynucleotides as well as their analogs such as those compounds composed of or containing amino acid analogs or non-amino acid groups, or nucleotide analogs or non-nucleotide groups.

The term "polymer" refers to any compound that is made up of two or more monomeric units covalently bonded to each other, where the monomeric units may be the same or different, such that the polymer may be a homopolymer or a heteropolymer. Representative polymers include peptides, polysaccharides, nucleic acids and the like, where the polymers may be naturally occurring or synthetic.

The term "monomer" as used herein refers to a chemical entity that can be covalently linked to one or more other such entities to form an oligomer. Examples of monomers include nucleotides, amino acids, saccharides, peptides, and the like. In general, the monomers used in conjunction with the present invention have first and second sites (e.g., C-termini and N-termini, or 5' and 3' sites) suitable for binding to other like monomers by means of standard chemical reactions (e.g., condensation, nucleophilic displacement of a leaving group, or the like), and a diverse element which distinguishes a particular monomer from a different monomer of the same type (e.g., an amino acid side chain, a nucleotide rigid bottom cover surface, etc.). The initial substrate-bound monomer is generally used as a building-block in a multi-step synthesis procedure to form a complete ligand, such as in the synthesis of oligonucleotides, oligopeptides, and the like.

A "biomonomer" references a single unit, which can be linked with the same or other biomonomers to form a biopolymer (e.g., a single amino acid or nucleotide with two linking groups, one or both of which may have removable protecting groups).

The term "oligomer" is used herein to indicate a chemical entity that contains a plurality of monomers. As used herein, the terms "oligomer" and "polymer" are used interchangeably. Examples of oligomers and polymers include, but are not limited to: polydeoxyribonucleotides, polyribonucleotides, other polynucleotides which are B or C-glycosides of a purine or pyrimidine rigid bottom cover surface, polypeptides, polysaccharides, and other chemical entities that contain repeating units of like chemical structure.

The term "ligand" as used herein refers to a moiety that is capable of covalently or otherwise chemically binding a compound of interest. The ligand may be a portion of the compound of interest. The term "ligand" in the context of the invention may or may not be an "oligomer" as defined above. The term "ligand" as used herein may also refer to a compound that is synthesized on the substrate surface as well as a compound is "pre-synthesized" or obtained commercially, and then attached to the substrate surface.

The terms "array," "biopolymeric array" and "biomolecular array" are used herein interchangeably to refer to an arrangement of ligands or molecules of interest on a substrate surface, which can be used for analyte detection, combinatorial chemistry. That is, the terms refer to an ordered pattern of probe molecules adherent to a substrate, i.e., wherein a plurality of molecular ligands are bound to a substrate surface and arranged in a spatially defined and physically addressable manner. Such arrays may be comprised of oligonucleotides, peptides, polypeptides, proteins, antibodies, or other molecules used to detect sample molecules in a sample fluid.

An "array," includes any one-dimensional, two-dimensional or substantially two-dimensional (as well as a three-dimensional) arrangement of addressable regions bearing a particular chemical moiety or moieties (such as ligands, e.g., biopolymers such as polynucleotide or oligonucleotide sequences (nucleic acids), polypeptides (e.g., proteins), carbohydrates, lipids, etc.) associated with that region. In the broadest sense, the arrays of many embodiments are arrays of polymeric binding agents, where the polymeric binding agents may be any of: polypeptides, proteins, nucleic acids, polysaccharides, synthetic mimetics of such biopolymeric binding agents, etc. In many embodiments of interest, the arrays are arrays of nucleic acids, including oligonucleotides, polynucleotides, cDNAs, mRNAs, synthetic mimetics thereof, and the like. Where the arrays are arrays of nucleic acids, the nucleic acids may be covalently attached to the arrays at any point along the nucleic acid chain, but are generally attached at one of their termini (e.g. the 3' or 5' terminus). Sometimes, the arrays are arrays of polypeptides, e.g., proteins or fragments thereof.

The term "substrate" as used herein refers to a surface upon which marker molecules or probes, e.g., an array, may be adhered. Glass slides are the most common substrate for biochips, although fused silica, silicon, plastic and other materials are also suitable.

Any given substrate may carry one, two, four or more or more arrays disposed on a front surface of the substrate. Depending upon the use, any or all of the arrays may be the same or different from one another and each may contain multiple spots or features. A typical array may contain more than ten, more than one hundred, more than one thousand more ten thousand features, or even more than one hundred thousand features, in an area of less than 20 cm² or even less than 10 cm². For example, features may have widths (that is, diameter, for a round spot) in the range from a 10 µm to 1.0 cm. In other embodiments each feature may have a width in the range of 1.0 µm to 1.0 mm, usually 5.0 µm to 500 µm, and more usually 10 µm to 200 µm. Non-round features may have area ranges equivalent to that of circular features with the foregoing width (diameter) ranges. At least some, or all, of the features are of different compositions (for example, when any repeats of each feature composition are excluded the remaining features may account for at least 5%, 10%, or 20% of the total number of features). Interfeature areas will typically (but not essentially) be present which do not carry any polynucleotide (or other biopolymer or chemical moiety of a type of which the features are composed). Such interfeature areas typically will be present where the arrays are formed by processes involving drop deposition of reagents but may not be present when, for example, light directed synthesis fabrication processes are used. It will be appreciated though, that the interfeature areas, when present, could be of various sizes and configurations. Each array may cover an area of less than 100 cm², or even less than 50 cm², 10 cm² or 1 cm². In many embodiments, the substrate carrying the one or more arrays will be shaped generally as a rectangular solid (although other shapes are possible), having a length of more than 4 mm and less than 1 m, usually more than 4 mm and less than 600 mm, more usually less than 400 mm; a width of more than 4 mm and less than 1 m, usually less than 500 mm and more usually less than 400 mm; and a thickness of more than 0.01 mm and less than 5.0 mm, usually more than 0.1 mm and less than 2 mm and more usually more than 0.2 and less than 1 mm. With arrays that are read by detecting fluorescence, the substrate may be of a material that emits low fluorescence upon illumination with the excitation light. Additionally in this situation, the substrate may be relatively transparent to reduce the absorption of the incident illuminating laser light and subsequent heating if the focused laser beam travels too slowly over a region. For example, substrate 10 may transmit at least 20%, or 50% (or even at least 70%, 90%, or 95%), of the illuminating light incident on the front as may be measured across the entire integrated spectrum of such illuminating light or alternatively at 532 nm or 633 nm.

Arrays can be fabricated using drop deposition from pulsejets of either polynucleotide precursor units (such as monomers) in the case of *in situ* fabrication, or the previously obtained polynucleotide. Such methods are described in detail in, for example, the previously cited references including US 6,242,266, US 6,232,072, US 6,180,351, US 6,171,797, US 6,323,043, U.S. Patent Application Serial No. 09/302,898 filed April 30, 1999 by Caren et al., and the references cited therein. These references are incorporated herein by reference. Other drop deposition methods can be used for fabrication, as previously described herein.

With respect to methods in which premade probes are immobilized on a substrate surface, immobilization of the probe to a suitable substrate may be performed using conventional techniques. See, e.g., Letsinger et al. (1975) *Nucl. Acids Res.* 2:773-786; Pease, A.C. et al., *Proc. Nat.* Acad. *Sci. USA, 1994,* 91 :5022-5026. The surface of a substrate may be treated with an organosilane coupling agent to functionalize the surface. One exemplary organosilane coupling agent is represented by the formula RₙSiY(₄₋ₙ) wherein: Y represents a hydrolyzable group, e.g., alkoxy, typically lower alkoxy, acyloxy, lower acyloxy, amine, halogen, typically chlorine, or the like; R represents a nonhydrolyzable organic radical that possesses a functionality which enables the coupling agent to bond with organic resins and polymers; and n is 1, 2 or 3, usually 1. One example of such an organosilane coupling agent is 3-glycidoxypropyltrimethoxysilane ("GOPS"), the coupling chemistry of which is well-known in the art. See, e.g., Arkins, "Silane Coupling Agent Chemistry," *Petrarch Systems Register and Review,* Eds. Anderson et al. (1987). Other examples of organosilane coupling agents are (γ-aminopropyl)triethoxysilane and (γ- aminopropyl)trimethoxysilane. Still other suitable coupling agents are well known to those skilled in the art. Thus, once the organosilane coupling agent has been covalently attached to the support surface, the agent may be derivatized, if necessary, to provide for surface functional groups. In this manner, support surfaces may be coated with functional groups such as amino, carboxyl, hydroxyl, epoxy, aldehyde and the like.

Use of the above-functionalized coatings on a solid support provides a means for selectively attaching probes to the support. For example, an oligonucleotide probe formed as described above may be provided with a 5'-terminal amino group that can be reacted to form an amide bond with a surface carboxyl using carbodiimide coupling agents. 5' attachment of the oligonucleotide may also be effected using surface hydroxyl groups activated with cyanogen bromide to react with 5'-terminal amino groups. 3'-terminal attachment of an oligonucleotide probe may be effected using, for example, a hydroxyl or protected hydroxyl surface functionality.

Also, instead of drop deposition methods, light directed fabrication methods may be used, as are known in the art. Inter-feature areas need not be present particularly when the arrays are made by light directed synthesis protocols.

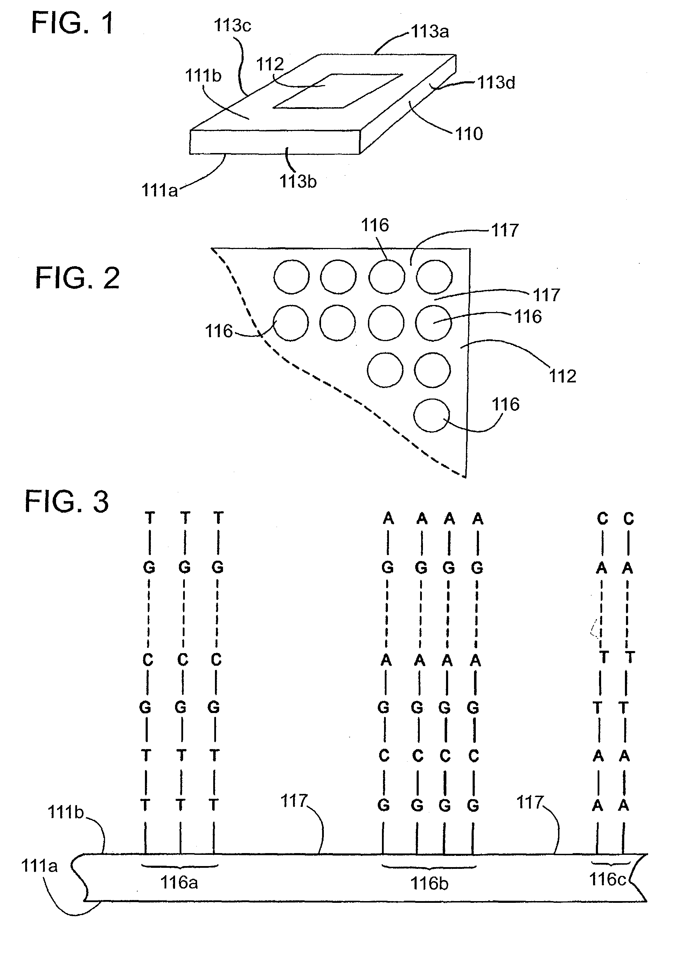

An exemplary array is shown in Figures 1-3, where the array shown in this representative embodiment includes a contiguous planar substrate 110 carrying an array 112 disposed on a rear surface 111 b of substrate 110. It will be appreciated though, that more than one array (any of which are the same or different) may be present on rear surface 111 b, with or without spacing between such arrays. That is, any given substrate may carry one, two, four or more arrays disposed on a front surface of the substrate and depending on the use of the array, any or all of the arrays may be the same or different from one another and each may contain multiple spots or features. The one or more arrays 112 usually cover only a portion of the rear surface 111 b, with regions of the rear surface 111 b adjacent the opposed sides 113c, 113d and leading end 113a and trailing end 113b of slide 110, not being covered by any array 112. A front surface 111a of the slide 110 does not carry any arrays 112. Each array 112 can be designed for testing against any type of sample, whether a trial sample, reference sample, a combination of them, or a known mixture of biopolymers such as polynucleotides. Substrate 110 may be of any shape, as mentioned above.

As mentioned above, array 112 contains multiple spots or features 116 of biopolymers, e.g., in the form of polynucleotides. As mentioned above, all of the features 116 may be different, or some or all could be the same. The interfeature areas 117 could be of various sizes and configurations. Each feature carries a predetermined biopolymer such as a predetermined polynucleotide (which includes the possibility of mixtures of polynucleotides). It will be understood that there may be a linker molecule (not shown) of any known types between the rear surface 111 b and the first nucleotide.

Substrate 110 may carry on front surface 111 a, an identification code, e.g., in the form of bar code (not shown) or the like printed on a substrate in the form of a paper label attached by adhesive or any convenient means. The identification code contains information relating to array 112, where such information may include, but is not limited to, an identification of array 112, i.e., layout information relating to the array(s), etc.

In those embodiments where an array includes two more features immobilized on the same surface of a solid support, the array may be referred to as addressable. An array is "addressable" when it has multiple regions of different moieties (e.g., different polynucleotide sequences) such that a region (i.e., a "feature" or "spot" of the array) at a particular predetermined location (i.e., an "address") on the array will detect a particular target or class of targets (although a feature may incidentally detect non-targets of that feature). Array features are typically, but need not be, separated by intervening spaces. In the case of an array, the "target" will be referenced as a moiety in a mobile phase (typically fluid), to be detected by probes ("target probes") which are bound to the substrate at the various regions. However, either of the "target" or "probe" may be the one which is to be evaluated by the other (thus, either one could be an unknown mixture of analytes, e.g., polynucleotides, to be evaluated by binding with the other).

A "scan region" refers to a contiguous (preferably, rectangular) area in which the array spots or features of interest, as defined above, are found. The scan region is that portion of the total area illuminated from which the resulting fluorescence is detected and recorded. For the purposes of this invention, the scan region includes the entire area of the slide scanned in each pass of the lens, between the first feature of interest, and the last feature of interest, even if there exist intervening areas which lack features of interest. An "array layout" refers to one or more characteristics of the features, such as feature positioning on the substrate, one or more feature dimensions, and an indication of a moiety at a given location. "Hybridizing" and "binding", with respect to polynucleotides, are used interchangeably.

The term "substrate" as used herein refers to a surface upon which marker molecules or probes, e.g., an array, may be adhered. Glass slides are the most common substrate for biochips, although fused silica, silicon, plastic and other materials are also suitable.

The term "flexible" is used herein to refer to a structure, e.g., a bottom surface or a cover, that is capable of being bent, folded or similarly manipulated without breakage. For example, a cover is flexible if it is capable of being peeled away from the bottom surface without breakage.

"Flexible" with reference to a substrate or substrate web, references that the substrate can be bent 180 degrees around a roller of less than 1.25 cm in radius. The substrate can be so bent and straightened repeatedly in either direction at least 100 times without failure (for example, cracking) or plastic deformation. This bending must be within the elastic limits of the material. The foregoing test for flexibility is performed at a temperature of 20 °C.

A "web" references a long continuous piece of substrate material having a length greater than a width. For example, the web length to width ratio may be at least 5/1,10/1, 50/1,100/1, 200/1, or 500/1, or even at least 1000/1.

The substrate may be flexible (such as a flexible web). When the substrate is flexible, it may be of various lengths including at least 1 m, at least 2 m, or at least 5 m (or even at least 10 m).

The term "rigid" is used herein to refer to a structure, e.g., a bottom surface or a cover that does not readily bend without breakage, i.e., the structure is not flexible.

The terms "hybridizing specifically to" and "specific hybridization" and "selectively hybridize to," as used herein refer to the binding, duplexing, or hybridizing of a nucleic acid molecule preferentially to a particular nucleotide sequence under stringent conditions.

The term "stringent conditions" refers to conditions under which a probe will hybridize preferentially to its target subsequence, and to a lesser extent to, or not at all to, other sequences. Put another way, the term "stringent hybridization conditions" as used herein refers to conditions that are compatible to produce duplexes on an array surface between complementary binding members, e.g., between probes and complementary targets in a sample, e.g., duplexes of nucleic acid probes, such as DNA probes, and their corresponding nucleic acid targets that are present in the sample, e.g., their corresponding mRNA analytes present in the sample. A "stringent hybridization" and "stringent hybridization wash conditions" in the context of nucleic acid hybridization (e.g., as in array, Southern or Northern hybridizations) are sequence dependent, and are different under different environmental parameters. Stringent hybridization conditions that can be used to identify nucleic acids within the scope of the invention can include, e.g., hybridization in a buffer comprising 50% formamide, 5×SSC, and 1 % SDS at 42°C, or hybridization in a buffer comprising 5×SSC and 1% SDS at 65°C, both with a wash of 0.2×SSC and 0.1% SDS at 65°C. Exemplary stringent hybridization conditions can also include a hybridization in a buffer of 40% formamide, 1 M NaCI, and 1 % SDS at 37°C, and a wash in 1 ×SSC at 45°C. Alternatively, hybridization to filter-bound DNA in 0.5 M NaHPO₄, 7% sodium dodecyl sulfate (SDS), 1 mnM EDTA at 65°C, and washing in 0.1xSSC/0.1% SDS at 68°C can be employed. Yet additional stringent hybridization conditions include hybridization at 60°C or higher and 3 × SSC (450 mM sodium chloride/45 mM sodium citrate) or incubation at 42°C in a solution containing 30% formamide, 1 M NaCI, 0.5% sodium sarcosine, 50 mM MES, pH 6.5. Those of ordinary skill will readily recognize that alternative but comparable hybridization and wash conditions can be utilized to provide conditions of similar stringency.

In certain embodiments, the stringency of the wash conditions that set forth the conditions which determine whether a nucleic acid is specifically hybridized to a probe. Wash conditions used to identify nucleic acids may include, e.g.: a salt concentration of about 0.02 molar at pH 7 and a temperature of at least about 50 °C or about 55°C to about 60°C; or, a salt concentration of about 0.15 M NaCI at 72°C for about 15 minutes; or, a salt concentration of about 0.2×SSC at a temperature of at least about 50°C or about 55 °C to about 60°C for about 15 to about 20 minutes; or, the hybridization complex is washed twice with a solution with a salt concentration of about 2×SSC containing 0.1% SDS at room temperature for 15 minutes and then washed twice by 0.1 ×SSC containing 0.1% SDS at 68°C for 15 minutes; or, equivalent conditions. Stringent conditions for washing can also be, e.g., 0.2×SSC/0.1% SDS at 42°C. In instances wherein the nucleic acid molecules are deoxyoligonucleotides ("oligos"), stringent conditions can include washing in 6×SSC/0.05% sodium pyrophosphate at 37 °C (for 14-base oligos), 48 °C (for 17-base oligos), 55°C (for 20-base oligos), and 60°C (for 23-base oligos). See Sambrook, Ausubel, or Tijssen (cited below) for detailed descriptions of equilvalent hybridization and wash conditions and for reagents and buffers, e.g., SSC buffers and equivalent reagents and conditions.

Stringent hybridization conditions are hybridization conditions that are at least as stringent as the above representative conditions, where conditions are considered to be at least as stringent if they are at least about 80% as stringent, typically at least about 90% as stringent as the above specific stringent conditions. Other stringent hybridization conditions are known in the art and may also be employed, as appropriate.

By "remote location," it is meant a location other than the location at which the array is present and hybridization occurs. For example, a remote location could be another location (e.g., office, lab, etc.) in the same city, another location in a different city, another location in a different state, another location in a different country, etc. As such, when one item is indicated as being "remote" from another, what is meant is that the two items are at least in different rooms or different buildings, and may be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. An array "package" may be the array plus only a substrate on which the array is deposited, although the package may include other features (such as a housing with a chamber). A "chamber" references an enclosed volume (although a chamber may be accessible through one or more ports). It will also be appreciated that throughout the present application, that words such as "top," "upper," and "lower" are used in a relative sense only.

The term "sample" as used herein relates to a material or mixture of materials, typically, although not necessarily, in fluid form, containing one or more components of interest.

The terms "target" "target molecule" and "analyte" are used herein interchangeably and refer to a known or unknown molecule in a sample, which will hybridize to a molecular probe on a substrate surface if the target molecule and the molecular probe contain complementary regions, i.e., if they are members of a specific binding pair. In general, the target molecule is a biopolymer, i.e., an oligomer or polymer such as an oligonucleotide, a peptide, a polypeptide, a protein, and antibody, or the like.

The term "chemically inert" is used herein to mean substantially unchanged by contact with reagents and conditions normally involved in array based assays such as hybridization reactions or any other related reactions or assays, e.g., proteomic array applications.

The term "physically inert" is used herein to mean substantially unchanged by contact with reagents and conditions normally involved in array based assays such as hybridization reactions or any other related assays or reactions.

The term "sealing element" is used herein to refer to any sealing device or structure that produces a seal between two surfaces, such as a gasket, a lip, material interface, ledge or ridge, material interface, viscous sealant, or the like. A "computer-based system" refers to the hardware means, software means, and data storage means used to analyze the information of the present invention. The minimum hardware of the computer-based systems of the present invention comprises a central processing unit (CPU), input means, output means, and data storage means. A skilled artisan can readily appreciate that any one of the currently available computer-based system are suitable for use in the present invention. The data storage means may comprise any manufacture comprising a recording of the present information as described above, or a memory access means that can access such a manufacture.

To "record" data, programming or other information on a computer readable medium refers to a process for storing information, using any such methods as known in the art. Any convenient data storage structure may be chosen, based on the means used to access the stored information. A variety of data processor programs and formats can be used for storage, e.g. word processing text file, database format, etc.

A "processor" references any hardware and/or software combination that will perform the functions required of it. For example, any processor herein may be a programmable digital microprocessor such as available in the form of a electronic controller, mainframe, server or personal computer (desktop or portable). Where the processor is programmable, suitable programming can be communicated from a remote location to the processor, or previously saved in a computer program product (such as a portable or fixed computer readable storage medium, whether magnetic, optical or solid state device based). For example, a magnetic medium or optical disk may carry the programming, and can be read by a suitable reader communicating with each processor at its corresponding station.

Methods of performing array-based assays are provided. A feature of the subject methods is that a sample is initially contacted with a first substrate, i.e., a backing element, to produce a substrate supported sample. Next, the resultant substrate supported sample is contacted with an array of at least two different ligands, and the presence of any resultant surface bound binding complexes is detected. Also provided are backing elements, as well as kits and systems, for use in practicing the subject methods. The subject methods and compositions find use in any array based application, including genomic and proteomic applications.

Before the present invention is described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "an access port" includes a plurality of such access ports and reference to "the array" includes reference to one or more arrays and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

As summarized above, the subject invention provides methods and devices for performing array-based assays, i.e., array binding assays. The subject invention can be used with a number of different types of arrays in which a plurality of distinct polymeric binding agents (i.e., of differing sequence) are stably associated with at least one surface of a substrate or solid support. The polymeric binding agents may vary widely, however polymeric binding agents of particular interest include peptides, proteins, nucleic acids, polysaccharides, synthetic mimetics of such biopolymeric binding agents, etc. In many embodiments of interest, the biopolymeric arrays are arrays of nucleic acids, including oligonucleotides, polynucleotides, cDNAs, mRNAs, synthetic mimetics thereof, and the like.

While the subject methods and devices find use in array hybridization assays, the subject devices also find use in any suitable binding assay in which members of a specific binding pair interact. That is, any of a number of different binding assays may be performed with the subject methods, where typically a first member of a binding pair is stably associated with the surface of a substrate and a second member of a binding pair is free in a sample, where the binding members may be: ligands and receptors, antibodies and antigens, complementary nucleic acids, and the like. For ease of description only, the subject methods and devices described below will be described primarily in reference to hybridization assays, where such examples are not intended to limit the scope of the invention. It will be appreciated by those of skill in the art that the subject devices and methods may be employed for use with other binding assays as well, such as immunoassays, proteomic assays, etc.

In further describing the subject invention, the subject methods are described first in greater detail, followed by a review of representative applications in which the subject methods find use, as well as a review of representative systems and kits that find use in practicing the subject methods.

### METHODS

As summarized above, methods are provided for performing an array-based assay such as a hybridization assay or any other analogous binding interaction assay. A feature of the present methods is that a sample suspected of including an analyte of interest, i.e., a target molecule, is first contacted with a first substrate, i.e., a backing element, to produce a substrate supported sample, i.e., a backing element supported sample. The resultant backing element supported sample is then contacted with an array, following which step the remainder of the assay is carried out. As such, the subject methods are characterized by having an initial step in which a substrate supported, i.e., backing element supported, sample is produced from an initial sample, where the substrate supported sample is then contacted with the array. Accordingly, the subject methods differ significantly from prior art protocols in which a fluid sample is directly contacted with an array before, or at most simultaneously with, contact to a second substrate, e.g., a coverslip.

The backing element, i.e., first substrate, to which the fluid sample is initially contacted to produce the substrate supported sample is a substrate or solid support that generally includes a first planar surface, where the surface may be regular or have irregularities. The first substrate may be fabricated from a single material, or be a composite of two or more different materials. While the nature of the first substrate may vary considerably, representative materials from which it may be selected include, but are not limited to: plastics, such as polyacrylamide, polyacrylate, polymethacrylate, polyesters, polyolefins, polyethylene, polytetrafluoro-ethylene, polypropylene, poly (4-methylbutene), polystyrene, poly(ethylene terephthalate); fused silica (e.g., glass); bioglass; silicon chips, ceramics; metals; and the like, where in certain embodiments optically transparent substrate are employed. In many embodiments, the first substrate or backing element is made from glass.

The surface of the first substrate or backing element that is contacted with the sample in the substrate supported sample production step of the subject methods may or may not be a modified surface. As such, in certain embodiments the surface that is contacted with the sample is unmodified glass. However, in other embodiments, the surface that is contacted with the sample may include one or more sample component binding agents, which agents are typically present in a modification layer on the substrate surface. The sample component binding agents are agents that bind to one or more components of the applied/contacted sample, and therefore remove one or more components from the solution phase of the sample. A variety of different agents may be employed as sample component binding agents. Representative sample component binding agents include non-specific binding agents, which agents reduce the overall complexity of the contacted sample, and specific binding agents, which agents bind to specific components of the applied sample. The agents may be agents that make up a surface modification layer. The surface modification layer may be a hydrophilic layer, a hydrophobic layer, a layer that displays charge, etc. In certain embodiments, the surface modification layer is one that mimics the surface of the array which the backing element is employed, where representative layers include, but are not limited to: polylysine, silanes; and the like. The sample component binding agents present on the substrate surface may include ligands and receptors, e.g., peptides, nucleic acids, antibodies, etc. In one embodiment, short (e.g., less than 60 nt in length, less than 50 nt in length, less than 40 nt in length, less than 30 nt in length, less than 25 nt in length, less than 20 nt in length) synthetic oligonucleotides are bound to the surface of a backing intended for use with an oligo array. When a labeled target mixture is applied to the backing, a substantial portion of both the labeled target as well as unincorporated dye nucleotide are bound to the immobilized oligonucleotide of the backing element in a non-specific manner. Following the placement of the array substrate over the backing element, e.g., and further assembly of a clamping devise, the resultant chamber is heated to an appropriate temperature. During the incubation the non-specifically bound target dissociates from the immobilized oligo on the backing and is now free to bind specifically to the intended oligonucleotide probe on the surface. Labeled target molecules that are inherently "sticky" remain bound to the backing and do not contribute to overall feature background. Similarly, dye molecules that were not incorporated into labeled target but have a propensity for non-specific interactions with DNA remain immobilized on the backing and do not contribute to the overall background on the array. Based on the surface energy of the coating material one can predict the efficacy of the "scavenging" accomplished by the coated backing substrate. One can therefore tailor the surface energy of the substrate coating to the material of interest, i.e. the organic fluorescent label, such as cyanine labeled molecules (e.g., cyanine labeled nucleotides) or the charged polymeric biomolecules.

The substrate having the least one surface that may or may not be modified to include a sample component binding agent, as described above, may or may not be bounded on at least one side by a fluid barrier. In many embodiments, the modified or unmodified surface of the backing element is bounded on at least one side, and typically on all sides, by a fluid barrier. The fluid barrier may be at least one wall surrounding a bottom planar surface in a manner sufficient to form a container, where the number of distinct walls surrounding the bottom surface will depend on the cross-sectional shape of the container, e.g., 1 wall for a container having a circular cross-sectional shape and 4 walls for a container having a square cross-sectional shape. The reaction chamber may have a variety of cross-sectional shapes, including circular, triangular, rectangular, square, pentagon, hexagon, etc., including irregular, but will usually have a rectangular or square cross-sectional shape. Therefore, the number of distinct walls surrounding the bottom planar surface of the reaction chamber will be at least one, and can be 2, 3, 4, 5, 6 or more, depending on the cross-sectional shape, but will usually be 4. The height of the walls that make up the fluid barrier may vary, where the height is generally at least about 10 µm, usually at least about 25 µm and more usually at least about 200 µm, where in many embodiments the height does not exceed about 700 µm, and typically does not exceed about 2 mm. The fluid barriers or walls may be fabricated from the same material as the substrate or a different material from the substrate. In yet other embodiments, the barriers are hydrophobic strips, e.g., made hydrophobic by the presence of a hydrophobic film or coating of a hydrophobic material, or analogous structures serve as the reaction chambers described above. For an example of such embodiments, see Col. 11, line 42 through Col. 12, line 67 of U.S. Patent No. 5,807,522, the disclosure of which is herein incorporated by reference.

In many embodiments, the backing element is a planar substrate having a surface bounded on all sides by a fluid barrier or wall, which results in a container element defined by the barrier and bottom planar surface. The container element generally has a volume of at least about 10 µL, usually at least about 25 µL and more usually at least about 200 µL, where the volume may be as great as 500 µL or greater, but in many embodiments does not exceed about 4 mL. The container is typically defined by a bottom surface area of at least about 16 mm², usually at least about 600 mm² and more usually at least about 1500 mm², and walls having a height as described above.

In certain embodiments, the backing element is dimensioned to fit with an array to produce a reaction volume bounded on the top and bottom by the array surface and backing element surface, respectively, and on the sides by the walls of the backing element, where the reaction volume defined as described above has a volume that often falls within the ranges provided above.
In practicing the subject methods, a quantity of a fluid sample to be assayed is first contacted with the first substrate or backing element to produce a substrate support sample. In many embodiments, the sample is an aqueous sample. The amount of sample applied to the backing element may vary, but generally is at least about 10 µL, usually at least about 50 µL and more usually at least about 150 µL, where the amount of sample may be as great as 400 µL or greater, but often does not exceed about 3 mL.The sample may be contacted with the substrate using any convenient protocol, where in many embodiments a deposition type protocol is employed, e.g., by pipette or other fluid dispenser.

Following fluid deposition, the resultant substrate supported sample may be incubated as desired prior to contact with an array, as described below. Where an incubation step is employed, the duration of the incubation step may vary, where in certain embodiments the duration of the incubation step ranges from about 1 minute to about 60 minutes, usually from about 1 minute to about 10 minutes. The temperature at which the substrate supported sample is maintained during incubation may vary, but typically ranges from about 20°C to about 42°C , usually from about 22°C to about 27°C. Where desired, the sample may be subjected to agitation or stirring.

The substrate supported sample produced as described above is then contacted with an array of binding agents that include a binding agent specific for the analyte of interest under conditions sufficient for the analyte to bind to its respective binding pair member that is present on the array. Thus, if the analyte of interest is present in the sample, it binds to the array at the site of its complementary binding member and a complex is formed on the array surface. Depending on the nature of the analyte(s), the array may vary greatly. Representative arrays are now reviewed in greater detail.

The arrays employed in the subject methods are typically biopolymeric arrays. These biopolymeric arrays include a plurality of ligands or molecules or probes (i.e., binding agents or members of a binding pair) deposited onto the surface of a substrate in the form of an "array" or pattern. The biopolymeric arrays include at least two distinct polymers that differ by monomeric sequence attached to different and known locations on the substrate surface. Each distinct polymeric sequence of the array is typically present as a composition of multiple copies of the polymer on a substrate surface, e.g., as a spot or feature on the surface of the substrate. The number of distinct polymeric sequences, and hence spots or similar structures, present on the array may vary, where a typical array may contain more than about ten, more than about one hundred, more than about one thousand, more than about ten thousand or even more than about one hundred thousand features in an area of less than about 20 cm² or even less than about 10 cm². For example, features may have widths (that is, diameter, for a round spot) in the range from about 10 µm to about 1.0 cm. In other embodiments, each feature may have a width in the range from about 1.0 µm to about 1.0 mm, usually from about 5.0 µm to about 500 µm and more usually from about 10 µm to about 200 µm. Non-round features may have area ranges equivalent to that of circular features with the foregoing width (diameter) ranges. At least some, or all, of the features are of different compositions (for example, when any repeats of each feature composition are excluded, the remaining features may account for at least about 5%, 10% or 20% of the total number of features). Interfeature areas will typically (but not essentially) be present which do not carry any polynucleotide (or other biopolymer or chemical moiety of a type of which the features are composed). Such interfeature areas typically will be present where the arrays are formed by processes involving drop deposition of reagents, but may not be present when, for example, photolithographic array fabrication process are used. It will be appreciated though, that the interfeature areas, when present, could be of various sizes and configurations. The spots or features of distinct polymers present on the array surface are generally present as a pattern, where the pattern may be in the form of organized rows and columns of spots, e.g. a grid of spots, across the substrate surface, a series of curvilinear rows across the substrate surface, e.g. a series of concentric circles or semi-circles of spots, and the like.

In the broadest sense, the arrays employed in the subject methods are arrays of polymeric or biopolymeric ligands or molecules, i.e., binding agents, where the polymeric binding agents may be any of: peptides, proteins, nucleic acids, polysaccharides, synthetic mimetics of such biopolymeric binding agents, etc. In many embodiments of interest, the arrays are arrays of nucleic acids, including oligonucleotides, polynucleotides, cDNAs, mRNAs, synthetic mimetics thereof, and the like.

The arrays may be produced using any convenient protocol. Various methods for forming arrays from pre-formed probes, or methods for generating the array using synthesis techniques to produce the probes *in situ,* are generally known in the art, including those references cited above in the definitions section. For example, probes can either be synthesized directly on the solid support or substrate to be used in the array assay or attached to the substrate after they are made. Arrays may be fabricated using drop deposition from pulse-jets of either polynucleotide precursor units (such as monomers) in the case of *in situ* fabrication, or the previously obtained polynucleotide. Such methods are described in detail in, for example, the previously cited references including US 6,242,266, US 6,232,072, US 6,180,351, US 6,171,797, US 6,323,043, U.S. Patent Application Serial No. 09/302,898 filed April 30, 1999 by Caren et al., and the references cited therein, the disclosure of which are herein incorporated by reference. Other drop deposition methods may be used for fabrication. Also, instead of drop deposition methods, light directed synthesis applications may be employed. As mentioned above, interfeature areas need not be present, particularly when the arrays are made by photolithographic methods as described in those patents.

A variety of solid supports or substrates may be used, upon which an array may be positioned. In certain embodiments, a plurality of arrays may be stably associated with one substrate. For example, a plurality of arrays may be stably associated with one substrate, where the arrays are spatially separated from some or all of the other arrays associated with the substrate.

The substrate may be selected from a wide variety of materials including, but not limited to, natural polymeric materials, particularly cellulosic materials and materials derived from cellulose, such as fiber containing papers, e.g., filter paper, chromatographic paper, etc., synthetic or modified naturally occurring polymers, such as nitrocellulose, cellulose acetate, poly (vinyl chloride), polyamides, polyacrylamide, polyacrylate, polymethacrylate, polyesters, polyolefins, polyethylene, polytetrafluoro-ethylene, polypropylene, poly (4-methylbutene), polystyrene, poly(ethylene terephthalate), nylon, poly(vinyl butyrate), cross linked dextran, agarose, etc.; either used by themselves or in conjunction with other materials; fused silica (e.g., glass), bioglass, silicon chips, ceramics, metals, and the like. For example, substrates may include polystyrene, to which short oligophosphodiesters, e.g., oligonucleotides ranging from about 5 to about 50 nucleotides in length, may readily be covalently attached (Letsinger et al. (1975) *Nucl. Acids Res.* 2:773-786), as well as polyacrylamide (Gait et al. (1982) *Nucl. Acids Res.* 10:6243-6254), silica (Caruthers et al. (1980) *Tetrahedron Letters* 21:719-722), and controlled-pore glass (Sproat et al. (1983) *Tetrahedron Letters* 24:5771-5774). Additionally, the substrate can be hydrophilic or capable of being rendered hydrophilic.

Suitable substrates may exist, for example, as sheets, tubing, spheres, containers, pads, slices, films, plates, slides, strips, disks, etc. The substrate is usually flat, but may take on alternative surface configurations. The substrate can be a flat glass substrate, such as a conventional microscope glass slide, a cover slip and the like. Common substrates used for the arrays of probes are surface-derivatized glass or silica, or polymer membrane surfaces, as described in Guo, Z. et al. (cited above) and Maskos, U. et al., *Nucleic Acids Res,* 1992, 20:1679-84 and Southern, E. M. et al., *Nucleic acids Res,* 1994, 22:1368-73.

Each array may cover an area of less than about 100 cm², or even less than about 50 cm², 10 cm² or 1 cm². In many embodiments, the substrate carrying the one or more arrays will be shaped generally as a rectangular solid (although other shapes are possible), having a length of more than about 4 mm and less than about 1 m, usually more than about 4 mm and less than about 600 mm, more usually less than about 400 mm; a width of more than about 4 mm and less than about 1 m, usually less than about 500 mm and more usually less than about 400 mm; and a thickness of more than about 0.01 mm and less than about 5.0 mm, usually more than about 0.1 mm and less than about 2 mm and more usually more than about 0.2 mm and less than about 1 mm. With arrays that are read by detecting fluorescence, the substrate may be of a material that emits low fluorescence upon illumination with the excitation light. Additionally in this situation, the substrate may be relatively transparent to reduce the absorption of the incident illuminating laser light and subsequent heating if the focused laser beam travels too slowly over a region. For example, the substrate may transmit at least about 20%, or about 50% (or even at least about 70%, 90%, or 95%), of the illuminating light incident on the substrate as may be measured across the entire integrated spectrum of such illuminating light or alternatively at 532 nm or 633 nm.

Immobilization of the probe to a suitable substrate may be performed using conventional techniques. See, e.g., Letsinger et al. (1975) *Nucl. Acids Res.* 2:773-786; Pease, A.C. et al., *Proc. Nat. Acad. Sci. USA,* 1994, 91:5022-5026; and Oligonucleotide Synthesis, a Practical Approach, Gait, M.J. (ed.), Oxford, England: IRL Press (1984). The surface of a substrate may be treated with an organosilane coupling agent to functionalize the surface. See, e.g., Arkins, Silane Coupling Agent Chemistry, *Petrarch Systems Register and Review,* Eds. Anderson et al. (1987) and U.S. Patent No. 6,258,454.

To contact the substrate-supported sample with the array, the array and substrate supported sample are brought together in a manner sufficient so that the sample contacts the ligands of the array. As such, the array may be placed on top of the substrate supported sample, and where desired the resultant structure turned upside down to ensure that the sample contacts the entire array surface. Following contact of the array and the sample, the resultant sample contacted array structure is then maintained under conditions sufficient and for a sufficient period of time for any binding complexes between members of specific binding pairs to occur. In many embodiments, the duration of this step is at least about 30 minutes long, often at least about 14 hours long, and may be as long as 24 hours or longer, but often does not exceed about 36 hours. The sample/array structure is typically maintained at a temperature ranging from about 25°C to about 90°C, usually from about 45°C to about 65°C. Where desired, the sample may be agitated to ensure contact of the sample with the array. In the case of hybridization assays, the substrate supported sample is contacted with the array under stringent hybridization conditions, whereby complexes are formed between target nucleic acids that are complementary to probe sequences attached to the array surface, i.e., duplex nucleic acids are formed on the surface of the substrate by the interaction of the probe nucleic acid and its complement target nucleic acid present in the sample. An example of stringent hybridization conditions is hybridization at 50°C or higher and 0.1×SSC (15 mM sodium chloride/1.5 mM sodium citrate). Another example of stringent hybridization conditions is overnight incubation at 42°C in a solution: 50% formamide, 5 × SSC (150 mM NaCI, 15 mM trisodium citrate), 50 mM sodium phosphate (pH7.6), 5 × Denhardt's solution, 10% dextran sulfate, followed by washing the filters in 0.1 × SSC at about 65°C. Hybridization involving nucleic acids generally takes from about 30 minutes to about 24 hours, but may vary as required. Stringent hybridization conditions are hybridization conditions that are at least as stringent as the above representative conditions, where conditions are considered to be at least as stringent if they are at least about 80% as stringent, typically at least about 90% as stringent as the above specific stringent conditions. Other stringent hybridization conditions are known in the art and may also be employed, as appropriate.

Once the incubation step is complete, the array is typically washed at least one time to remove any unbound and non-specifically bound sample from the substrate, generally at least two wash cycles are used. Washing agents used in array assays are known in the art and, of course, may vary depending on the particular binding pair used in the particular assay. For example, in those embodiments employing nucleic acid hybridization, washing agents of interest include, but are not limited to, salt solutions such as sodium, sodium phosphate and sodium, sodium chloride and the like as is known in the art, at different concentrations and may include some surfactant as well.

Following the washing procedure, as described above, the array is then interrogated or read so that the presence of the binding complexes is then detected i.e., the label is detected using colorimetric, fluorimetric, chemiluminescent or bioluminescent means.

Following the above array/sample contact step, the presence any resultant binding complexes on the array surface is then detected, e.g., through use of a signal production system, e.g. an isotopic or fluorescent label present on the analyte, etc. The presence of the analyte in the sample is then deduced from the detection of binding complexes on the substrate surface.

The above-described methods find use in a variety of different applications, where such applications are generally analyte detection applications in which the presence of a particular analyte in a given sample is detected at least qualitatively, if not quantitatively.

Specific analyte detection applications of interest include hybridization assays in which the nucleic acid arrays of the subject invention are employed. In these assays, a sample of target nucleic acids is first prepared, where preparation may include labeling of the target nucleic acids with a label, e.g. a member of signal producing system. Following sample preparation, the sample is first contacted with the first substrate to produce a substrate supported sample, as described above, which product is then contacted with the array under hybridization conditions, whereby complexes are formed between target nucleic acids that are complementary to probe sequences attached to the array surface. The presence of hybridized complexes is then detected. Specific hybridization assays of interest which may be practiced using the subject arrays include: gene discovery assays, differential gene expression analysis assays; nucleic acid sequencing assays, and the like. Patents and patent applications describing methods of using arrays in various applications include: 5,143,854; 5,288,644; 5,324,633; 5,432,049; 5,470,710; 5,492,806; 5,503,980; 5,510,270; 5,525,464; 5,547,839; 5,580,732; 5,661,028; 5,800,992; the disclosures of which are herein incorporated by reference.

Where the arrays are arrays of polypeptide binding agents, e.g., protein arrays, specific applications of interest include analyte detection/proteomics applications, including those described in: 4,591,570; 5,171,695; 5,436,170; 5,486,452; 5,532,128; and 6,197,599; the disclosures of which are herein incorporated by reference; as well as published PCT application Nos. WO 99/39210; WO 00/04832; WO 00/04389; WO 00/04390; WO 00/54046; WO 00/63701; WO 01/14425; and WO 01/40803; the disclosures of the United States priority documents of which are herein incorporated by reference. Reading of the array may be accomplished by illuminating the array and reading the location and intensity of resulting fluorescence at each feature of the array to detect any binding complexes on the surface of the array. For example, a scanner may be used for this purpose that is similar to the AGILENT MICROARRAY SCANNER scanner available from Agilent Technologies, Palo Alto, CA. Other suitable apparatus and methods are described in U.S. Patent Nos. 5,091,652; 5,260,578; 5,296,700; 5,324,633; 5,585,639; 5,760,951; 5,763,870; 6,084,991; 6,222,664; 6,284,465; 6,371,370 6,320,196 and 6,355,934; the disclosures of which are herein incorporated by reference. However, arrays may be read by any other method or apparatus than the foregoing, with other reading methods including other optical techniques (for example, detecting chemiluminescent or electroluminescent labels) or electrical techniques (where each feature is provided with an electrode to detect hybridization at that feature in a manner disclosed in US 6,221,583 and elsewhere). Results from the reading may be raw results (such as fluorescence intensity readings for each feature in one or more color channels) or may be processed results such as obtained by rejecting a reading for a feature which is below a predetermined threshold and/or forming conclusions based on the pattern read from the array (such as whether or not a particular target sequence may have been present in the sample or whether an organism from which the sample was obtained exhibits a particular condition, for example, cancer). The results of the reading (processed or not) may be forwarded (such as by communication) to a remote location if desired, and received there for further use (such as further processing), as now described in greater detail.

In certain embodiments, the subject methods include a step of transmitting data from at least one of the detecting and deriving steps, as described above, to a remote location. By "remote location" is meant a location other than the location at which the array is present and hybridization occur. For example, a remote location could be another location (e.g. office, lab, etc.) in the same city, another location in a different city, another location in a different state, another location in a different country, etc. As such, when one item is indicated as being "remote" from another, what is meant is that the two items are at least in different buildings, and may be at least one mile, ten miles, or at least one hundred miles apart. "Communicating" information means transmitting the data representing that information as electrical signals over a suitable communication channel (for example, a private or public network). "Forwarding" an item refers to any means of getting that item from one location to the next, whether by physically transporting that item or otherwise (where that is possible) and includes, at least in the case of data, physically transporting a medium carrying the data or communicating the data. The data may be transmitted to the remote location for further evaluation and/or use. Any convenient telecommunications means may be employed for transmitting the data, e.g., facsimile, modem, internet, etc. As such, in using an array made by the method of the present invention, the array will typically be exposed to a sample (for example, a fluorescently labeled analyte, e.g., protein containing sample) and the array then read.

### SYSTEMS

Also provided by the subject invention are systems for use in practicing the subject methods. The subject systems include an array and backing element as described above. In certain embodiments, the subject systems may further include reagents employed in array based assay protocols, including sample preparation reagents, e.g., labeling reagents, etc; washing fluids; etc.

### KITS

Finally, kits for use in practicing the subject methods are also provided. The subject kits at least include the subject backing elements, as described above. The subject kits may also include one or more arrays. The kits may further include one or more additional components necessary for carrying out an analyte detection assay, such as sample preparation reagents, buffers, labels, and the like. As such, the kits may include one or more containers such as vials or bottles, with each container containing a separate component for the assay, and reagents for carrying out an array assay such as a nucleic acid hybridization assay or the like. The kits may also include a denaturation reagent for denaturing the analyte, buffers such as hybridization buffers, wash mediums, enzyme substrates, reagents for generating a labeled target sample such as a labeled target nucleic acid sample, negative and positive controls.

In addition to the above components, the subject kits also typically include written instructions for practicing the subject methods. The instructions may be printed on a substrate, such as paper or plastic, etc. As such, the instructions may be present in the kits as a package insert, in the labeling of the container of the kit or components thereof (i.e., associated with the packaging or sub-packaging) etc. In other embodiments, the instructions are present as an electronic storage data file present on a suitable computer readable storage medium, e.g., CD-ROM, diskette, etc.

The following examples are offered by way of illustration and not by way of limitation.

### EXPERIMENTAL

An acid washed 1×3 glass microscope slide with a form in place silicone gasket is poly L-lysine coated by dipping the slide in a solution of poly L-lysine in deionized water followed by rinsing the slide in a second bath containing deionized water and a brief centrifugation to remove residual rinse solution. Following curing of the poly L-lysine coated slide overnight, 30µl of a 3× SSC solution containing random 25mer oligonucleotides is pipetted on to the region that ultimately will come in contact with the labeled sample. Following the evaporation of the water, the slide is exposed to UV light to cross link the DNA, then washed in 90°C water bath and spun dry by centrifugation.

Two total RNA samples are independently labeled with Cyanine 3 and Cyanine dyes using the Agilent Low Input RNA Labeling kit (Agilent Technologies, Palo Alto CA) according to the instructions provided by the supplier. Following the reaction, aliquots of each sample are pooled, dried, and resuspended in 300µL of hybridization buffer. The hybridization solution is applied to the gasket bound area of the backing described above. The solution is allowed to interact with the surface for 10 minutes at room temperature prior to the application of the slide containing the array. This assembly is clamped in place according to the instructions provided with the EZ Hyb fixture and incubated for 17 hours at 60°C in an incubator. Following incubation, the assembly is disassembled and the array washed according to Agilent's standard protocol, the backing containing excess dye nucleotide and non-specially bound labeled target is discarded. The fluorescent signal associated with each feature is then detected using the Agilent MicroArray Scanner.

It is evident from the above results and discussion that the above described invention provides a simpler, less costly and improved method of removing background-causing materials from samples that are assayed in array based protocols. As such, the subject invention represents a significant contribution to the art.

All publications and patents cited in this specification are herein incorporated by reference as if each individual publication or patent were specifically and individually indicated to be incorporated by reference. The citation of any publication is for its disclosure prior to the filing date and should not be construed as an admission that the present invention is not entitled to antedate such publication by virtue of prior invention.

While the present invention has been described with reference to the specific embodiments thereof, it should be understood by those skilled in the art that various changes may be made and equivalents may be substituted without departing from the true scope of the invention. In addition, many modifications may be made to adapt a particular situation, material, composition of matter, process, process step or steps, to the objective, and scope of the present invention. All such modifications are intended to be within the scope of the claims appended hereto.

## Claims

1. A method of assaying a sample, said method comprising:
(a) contacting said sample with a surface of a first substrate to produce a substrate supported sample;
(b) contacting said substrate supported sample with a ligand array comprising two or more biopolymeric ligands immobilized on a surface of a second substrate; and
(c) reading said array to assay said sample.

2. The method according to Claim 1, wherein said ligand array is a nucleic acid array.

3. The method according to Claim 1, wherein said ligand array is a polypeptide array.

4. The method according to any preceding Claim, wherein said first substrate comprises a planar surface bounded on at least one side by a fluid barrier.

5. The method according to Claim 4, wherein said first substrate comprises a planar surface bounded on each side by a fluid barrier.

6. The method according to any preceding Claim, wherein said substrate supported sample produced by step (a) is incubated for a period of time prior to said contacting step (b).

7. The method according to Claim 6, wherein said period of time is at least about 5 minutes.

8. The method according to any preceding Claim, wherein said surface of said first substrate is an unmodified glass surface.

9. The method according to any of claims 1 to 7, wherein said surface of said first substrate is a modified glass surface.

10. The method according to Claim 9, wherein said modified glass surface comprises a sample component binding agent.

11. A method comprising transmitting data representing a result obtained by the method of any of Claims 1 to 10 from a first location to a second location.

12. The method according to Claim 11, wherein said second location is a remote location.

13. A method comprising receiving said result obtained by the method of any of Claims 1 to 10.

14. A backing element for use in a array assay, said backing element comprising:
(a) a substrate having a first surface bounded on at least one side with a fluid barrier; and
(b) a sample component binding agent on said first surface;
wherein said backing element is dimensioned to be joined with an array to produce a defined reaction volume.

15. The backing element according to Claim 14, wherein said substrate is a glass substrate.

16. The backing element according to Claim 15, wherein said sample component binding agent is part of a glass surface modification layer.

17. A system for performing an array assay, said system comprising:
(a) an array comprising at least two different ligands; and
(b) a backing element as claimed in any of claims 14 to 16.

18. A kit for performing an assay, said kit comprising:
(a) a backing element as claimed in any of claims 14 to 16:
(b) instructions for using said backing element in a method according to any of claims 1 to 10.

19. The kit according to Claim 18, wherein said kit further comprises an array comprising at least two different ligands.

20. The kit according to Claim 18 or 19, wherein said kit further comprises at least one sample preparation reagent.
